(19) Europäisches Patentamt
European Patent Office
Office européen des brevets

(11) Veröffentlichungsnummer: **0 207 004**
**A2**

(12) # EUROPÄISCHE PATENTANMELDUNG

(21) Anmeldenummer: **86810286.4**

(22) Anmeldetag: **23.06.86**

(51) Int. Cl.[4]: **C 07 D 417/04**, C 07 D 417/14, A 01 N 43/82

(30) Priorität: **28.06.85 CH 2752/85**
**23.08.85 CH 3640/85**
**27.05.86 CH 2131/86**

(43) Veröffentlichungstag der Anmeldung: **30.12.86**
**Patentblatt 86/52**

(84) Benannte Vertragsstaaten: **AT BE CH DE FR GB IT LI NL**

(71) Anmelder: **CIBA-GEIGY AG, Klybeckstrasse 141, CH-4002 Basel (CH)**

(72) Erfinder: **Farooq, Saleem, Dr., Kirchackerstrasse 27, CH-4422 Arisdorf (CH)**
Erfinder: **Ehrenfreund, Josef, Dr., Amselstrasse 11, CH-4123 Allschwil (CH)**
Erfinder: **Waespe, Hans-Rudolf, Dr., Feldstrasse 86, CH-4123 Allschwil (CH)**

(54) **Substituierte 4,5-Dihydro-1,3,4-thiadiazole.**

(57) Neue gegebenenfalls substituierte 2-Phenyl-5-Pyridyl-4,5-dihydro-1,3,4-thiadiazole der Formel

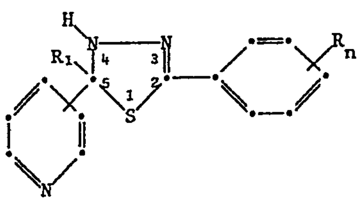

worin

$R_1$ Wasserstoff oder $C_1$-$C_4$-Alkyl;

R Wasserstoff, $C_1$-$C_4$-Alkyl, Halogen, Nitro, Cyano, $C_1$-$C_4$-Alkoxy, $C_1$-$C_4$-Alkylthio, $C_1$-$C_4$-Halogenalkyl mit 1 bis 9 Halogenatomen, $C_1$-$C_4$-Halogenalkoxy mit 1 bis 9 Halogenatomen, $C_1$-$C_4$-Halogenalkylthio mit 1 bis 9 Halogenatomen, Phenyl, substituiertes Phenyl, Phenylalkyl, substituiertes Phenylalkyl, Phenoxy, substituiertes Phenoxy, Phenylthio, substituiertes Phenylthio, Pyridyloxy oder substituiertes Pyridyloxy bedeuten und

n für eine Zahl von 1 bis 5 steht; sowie deren Salze und optischen Isomeren; Verfahren zur Herstellung dieser Verbindungen sowie diese enthaltende Mittel zur Verwendung in der Schädlingsbekämpfung, insbesondere zur Bekämpfung von Pflanzen und Tiere befallenden Insekten. Die neuen Verbindungen weisen insbesondere gute Wirksamkeit gegen pflanzenschädigende saugende Insekten auf.

EP 0 207 004 A2

CIBA-GEIGY AG
Basel (Schweiz)

5-15409/1-3

Substituierte 4,5-Dihydro-1,3,4-thiadiazole

Die vorliegende Erfindung betrifft neue, gegebenenfalls substituierte 2-Phenyl-5-pyridyl-4,5-dihydro-1,3,4-thiadiazole, Verfahren zu ihrer Herstellung und ihre Verwendung in der Schädlingsbekämpfung.

Die vorliegende Erfindung bezieht sich auf neue Verbindungen der Formel I

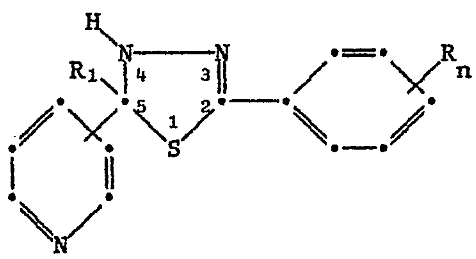

(I),

worin

$R_1$ Wasserstoff oder $C_1$-$C_4$-Alkyl;

R Wasserstoff, $C_1$-$C_4$-Alkyl, Halogen, Nitro, Cyano, $C_1$-$C_4$-Alkoxy, $C_1$-$C_4$-Alkylthio, $C_1$-$C_4$-Halogenalkyl mit 1 bis 9 Halogenatomen, $C_1$-$C_4$-Halogenalkoxy mit 1 bis 9 Halogenatomen, $C_1$-$C_4$-Halogenalkylthio mit 1 bis 9 Halogenatomen, Phenyl, substituiertes Phenyl, Phenylalkyl, substituiertes Phenylalkyl, Phenoxy, substituiertes Phenoxy, Phenylthio, substituiertes Phenylthio, Pyridyloxy oder substituiertes Pyridyloxy bedeuten und

n für eine Zahl von 1 bis 5 steht; sowie die Salze und optischen Isomeren einer Verbindung der Formel I.

Bevorzugt werden erfindungsgemäss Verbindungen der Formel I, worin

$R_1$ Wasserstoff oder $C_1$-$C_4$-Alkyl;

R Wasserstoff, $C_1$-$C_4$-Alkyl, Halogen, Nitro, Cyano, $C_1$-$C_4$-Alkoxy, $C_1$-$C_4$-Alkylthio, $C_1$-$C_4$-Halogenalkyl mit 1 bis 9 Halogenatomen, $C_1$-$C_4$-Halogenalkoxy mit 1 bis 9 Halogenatomen oder $C_1$-$C_4$-Halogenalkylthio mit 1 bis 9 Halogenatomen bedeuten und

n für eine Zahl von 1 bis 5 steht.

Weiterhin bevorzugt werden erfindungsgemäss Verbindungen der Formel I, worin n 1 oder 2 ist.

Falls R in den Verbindungen der Formel I die Bedeutung substituiertes Phenyl, substituiertes Phenylalkyl, substituiertes Phenoxy, substituiertes Phenylthio oder substituiertes Pyridyloxy hat, wobei diese Reste 1- bis 3-fach, vorzugsweise 1-fach, substituiert sein können, kommen als Substituenten unabhängig voneinander vorzugsweise die folgenden in Betracht: $C_1$-$C_4$-Alkyl, Halogen, vorzugsweise Fluor oder Chlor, Nitro, Cyano, $C_1$-$C_4$-Alkoxy, $C_1$-$C_4$-Alkylthio, $C_1$-$C_4$-Halogenalkyl mit 1 bis 9 Halogenatomen, $C_1$-$C_4$-Halogenalkoxy mit 1 bis 9 Halogenatomen und $C_1$-$C_4$-Halogenalkylthio mit 1 bis 9 Halogenatomen.

Von besonderem Interesse sind solche erfindungsgemässen Verbindungen der Formel Ia)

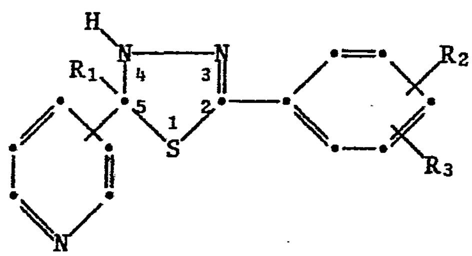

(Ia),

worin

$R_1$ Wasserstoff oder $C_1$-$C_4$-Alkyl;

$R_2$ und $R_3$ unabhängig voneinander Wasserstoff, Halogen, $C_1$-$C_4$-Alkyl, Trifluormethyl, $C_1$-$C_4$-Alkoxy oder Nitro

bedeuten; sowie die Salze einer Verbindung der Formel Ia), und insbesondere solche Verbindungen der Formel Ia), worin
$R_1$ Wasserstoff, Methyl oder Aethyl;
$R_2$ und $R_3$ unabhängig voneinander Wasserstoff oder in 2- und/oder 4-Stellung befindliches Halogen
bedeuten.

Bevorzugt sind daneben solche erfindungsgemässen Verbindungen der Formeln I bzw. Ia), worin $R_1$ und/oder $R_3$ Wasserstoff bedeuten.

Aufgrund ihrer biologischen Wirkung sind diejenigen Verbindungen der Formel I bzw. Ia besonders interessant, worin der Pyridylrest mit seiner 3-Stellung an die 5-Stellung des 4,5-Dihydro-1,3,4-thiadiazolringes gebunden ist.

Der vorliegende Erfindungsgegenstand umfasst auch die Salze, insbesondere die pflanzenphysiologisch unbedenklichen Salze, der Verbindungen der Formeln I bzw. Ia). Als derartige Salze mit organischen und anorganischen Säuren kommen z.B. die folgenden in Betracht: Chloride, Bromide, Jodide, Sulfate, Hydrogensulfate, Chlorate, Perchlorate, Rhodanide, Nitrate, Phosphate, Hydrogenphosphate, Tetrafluorborate, Formiate, Acetate, Trichloracetate, Trifluoracetate, Phenylsulfonate, Oxalate, Malonate, Succinate, Malate, Tartrate oder Citrate.

Der vorliegende Erfindungsgegenstand umfasst wieterhin die optischen Isomeren der Verbindungen der Formeln I bzw. Ia), die aus den racemischen Formen in an sich bekannter Weise mit Hilfe der üblichen Trennungsmethoden erhalten werden können.

Die Verbindungen der Formel I bzw. Ia) können in an sich bekannter Weise (vgl. D.M. Evans et al., J.Chem.Soc., Chem. commun. <u>1982</u>, 188; K.N. Zelenin et al., Khim. Geterotsikl. Soedin, <u>1982 No. 7</u>, 904) hergestellt werden durch Umsetzung einer Verbindung der Formel II

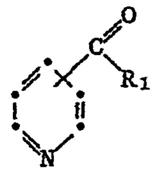

(II)

mit einer Verbindung der Formel III

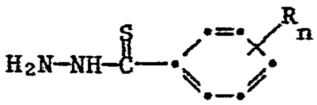

(III),

wobei in den Verbindungen II und III $R_1$, R und n die vorstehend unter Formel I angegebenen Bedeutungen haben (Verfahren a).

Die Verbindungen der Formel I bzw. Ia) können weiterhin in neuartiger Weise dadurch hergestellt werden, dass man eine Verbindung der Formel IV

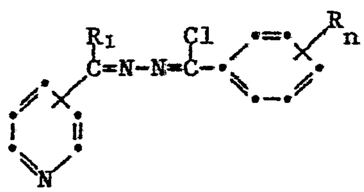

(IV),

worin $R_1$, $R_2$ und n die vorstehend unter Formel I angegebenen Bedeutungen haben, mit einem Sulfid umsetzt (Verfahren b).

Gegebenenfalls kann man eine wie vorstehend angegeben erhaltene Verbindung der Formeln I bzw. Ia) in an sich bekannter Weise in eines ihrer Salze überführen.

Das zu den erfindungsgemässen Verbindungen der Formeln I bzw. Ia) führende Verfahren a) wird vorzugsweise in einem Lösungsmittel vorgenommen. Geeignete Lösungsmittel sind z.B. aromatische Kohlenwasserstoffe, wie Benzol, Toluol und Xylol; Ketone, wie Aceton, Cyclohexanon und Methyläthylketon; Aether, wie Tetrahydrofuran, Dioxan und Diäthyläther; halogenierte Kohlenwasserstoffe, wie Chloroform, Tetrachlorkohlenstoff und Chlorbenzol; Alkohole, wie Aethanol und Propanol; Ester aliphatischer Säuren, wie Aethylacetat; aliphatische Amide, wie Dimethylformamid und Dimethylacetamid;

Dimethylsulfoxid und andere Lösungsmittel, die die Reaktion nicht beeinträchtigen. Diese Lösungsmittel können auch als Gemische verwendet werden. Die Reaktionstemperatur kann in einem weiten Bereich von -10 bis +200°C liegen. Bevorzugt wird ein Temperaturbereich von Raumtemperatur bis etwa 150°C.

Verfahren b) wird vorzugsweise in einem polaren Lösungsmittel, wie Wasser, einem Alkohol, z.B. Methanol oder Aethanol, durchgeführt. Die Ringschlussreaktion wird mit einem Sulfid, vorzugsweise einem löslichen Metallsufid, wie Natrium- oder Kaliumsulfid, oder einem entsprechenden Hydrogensulfid vorgenommen, wobei ein alkalisches Milieu bevorzugt wird. Die Reaktionstemperatur kann im Bereich von -15 bis +100°C liegen. Bevorzugt wird ein Temperaturbereich von -10 bis +50°C, z.B. Raumtemperatur.

Die als Ausgangsstoffe verwendeten Pyridin-carbonylverbindungen der Formel II und Thiobenzoesäurehydrazide der Formel III sind bekannt und können analog bekannter Verfahren erhalten werden. Die substituierten 2,3-Diazabutadiene der Formel IV, die als neue Verbindungen ebenfalls einen Gegenstand der vorliegenden Erfindung bilden, sind analog bekannter Arbeitsweisen (vgl. W.T. Flowers et al, J.Chem.Soc., Perkin. Trans., 1 (2), 349, 1981) wie folgt zugänglich, wobei $R_1$, R und n die vorstehend angegebenen Bedeutungen haben:

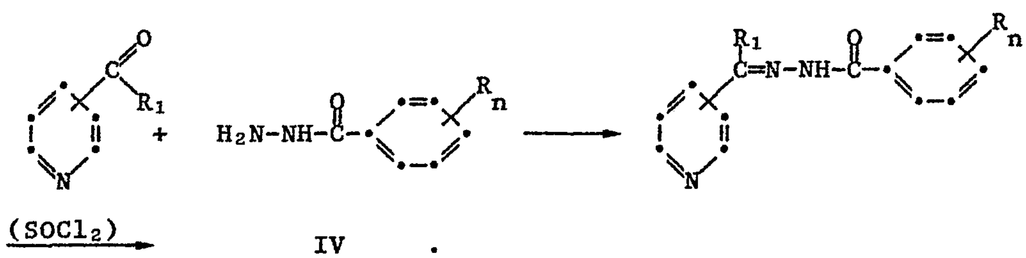


IV .

Es wurde gefunden, dass die erfindungsgemässen Verbindungen der Formeln I bzw. Ia) bei guter Pflanzenverträglichkeit und geringer Warmblütertoxizität ausgezeichnete Wirksamkeit als Schädlingsbekämpfungsmittel aufweisen. Sie eignen sich vor allem zur Bekämpfung von Pflanzen und Tieren befallenden Schädlingen.

Insbesondere eignen sich die Verbindungen der Formeln I bzw. Ia) zur Bekämpfung von Insekten der Ordnungen Lepidoptera, Coleoptera, Homoptera, Heteroptera, Diptera, Thysanoptera, Orthoptera, Anoplura, Siphonaptera, Mallophaga, Thysanura, Isoptera, Psocoptera und Hymenoptera sowie von Vertretern der Ordnung Akarina.

Die gute pestizide Wirkung der erfindungsgemässen Verbindungen entspricht einer Abtötungsrate (Mortalität) von mindestens 50-60 % der erwähnten Schädlinge.

Mit Hilfe der erfindungsgemässen Verbindungen der Formel I bzw. Ia können vor allem pflanzenschädigende Insekten, speziell pflanzenschädigende Insekten in Zier- und Nutzpflanzungen, insbesondere in Baumwollkulturen, Gemüsekulturen, Reiskulturen und Obstkulturen bekämpft werden. In diesem Zusammenhang ist hervorzuheben, dass die genannten Verbindungen sich sowohl durch eine stark ausgeprägte systemische, vor allem aber durch Kontakt-Wirkung gegen saugende Insekten, insbesondere gegen Insekten der Familie Aphididae (wie z.B. Aphis fabae, Aphis craccivora und Myzus persicae), welche sich mit herkömmlichen Mitteln nur schwierig bekämpfen lassen, auszeichnen.

Die Verbindungen der Formeln I bzw. Ia) zeichnen sich weiterhin durch eine gute Wirkung gegen larvale Insektenstadien und Nymphen, speziell von fressenden Schadinsekten, aus. Insbesondere können die Verbindungen der Formel I mit ausgezeichnetem Erfolg gegen pflanzenschädigende Zikaden, speziell in Reis-Kulturen, eingesetzt werden. Die Verbindungen eignen sich ferner zur Bekämpfung von Ektoparasiten, z.B. Lucilia sericata, sowie von Zecken an Haus- und Nutztieren, z.B. durch Tier-, Stall- und Weidebehandlung.

Die Wirkung der erfindungsgemässen Verbindungen bzw. der sie enthaltenden Mittel lässt sich durch Zusatz von anderen Insektiziden und/oder Akariziden wesentlich verbreitern und an gegebene Umstände

anpassen. Als Zusätze kommen z.B. Vertreter der folgenden Wirkstoffklassen in Betracht: Organische Phosphorverbindungen, Nitrophenole und Derivate, Formamidine, Harnstoffe, Carbamate, Pyrethroide, chlorierte Kohlenwasserstoffe und Bacillus thuringiensis-Präparate.

Die Verbindungen der Formeln I bzw. Ia) werden in unveränderter Form oder vorzugsweise zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren, wie Versprühen, Vernebeln, Verstäuben, Verstreuen oder Giessen, werden ebenso wie die Mittel den angestrebten Zielen und den gegebenen Verhältnissen entsprechend gewählt.

Die Formulierung, d.h. die den Wirkstoff, bzw. Kombinationen dieser Wirkstoffe mit anderen Insektiziden oder Akariziden, und gegebenenfalls einen festen oder flüssigen Zusatzstoff enthaltenden Mittel, Zubereitungen oder Zusammensetzungen, werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester, wie Dibutyl- oder Dioctylphthalat, aliphatische Kohlenwasserstoffe, wie Cyclohexan, Paraffine, Alkohole und Glykole sowie deren Aether und Ester, wie Aethanol, Aethylenglykol, Aethylenglykolmonomethyl- oder -äthyläther, Ketone, wie Cyclohexanon, stark polare Lösungsmittel, wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl, oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäuren oder hochdisperse saugfähige Polymerisate zugesetzt werden. Als gekörnte, adsorptive Granulatträger kommen poröse Typen, wie z.B. Bimsstein, Ziegelbruch, Sepiolit oder Bentonit, als nicht sorptive Trägermaterialien z.B. Calcit oder Sand in Frage. Darüberhinaus kann eine Vielzahl von granulierten Materialien anorganischer oder organischer Natur, wie insbesondere Dolomit oder zerkleinerte Pflanzenrückstände, verwendet werden.

Als oberflächenaktive Verbindungen kommen je nach Art des zu formulierenden Wirkstoffes der Formeln I bzw. Ia) oder der Kombinationen dieser Wirkstoffe mit andern Insektiziden oder Akariziden nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen eignen sich die Alkali-, Erdalkali- oder gegebenenfalls substituierten Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Tallöl gewonnen werden können. Ferner sind als Tenside auch die Fettsäuremethyl-taurin-salze sowie modifizierte und nicht modifizierte Phospholipide zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen im allgemeinen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2 Sulfonsäuregruppen und einen Fettsäurerest mit etwa 8-22 C-Atomen. Alkylarylsulfonate sind z.B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes. Ferner kommen auch entsprechende Phosphate, wie z.B. Salze des Phosphorsäureesters eines p-Nonylphenol-(4-14)-Aethylenoxid-Adduktes, in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können. Weiterhin geeignete nichtionische Tenside sind die wasserlöslichen 20 bis 250 Aethylenglykoläthergruppen und 10 bis 100 Propylenglykoläthergruppen enthaltenden Polyäthylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykoleinheiten.

Als Beispiele nichtionischer Tenside seien Nonylphenolpolyäthoxyäthanole, Ricinusölpolyglykoläther, Polypropylen-Polyäthylenoxidaddukte, Tributylphenoxypolyäthoxyäthanol, Polyäthylenglykol und

Octylphenoxypolyäthoxyäthanol erwähnt. Ferner kommen auch Fettsäureester von Polyoxyäthylensorbitan, wie das Polyoxyäthylensorbitantrioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Diese Salze liegen vorzugsweise als Halogenide, Methylsulfate oder Aethylsulfate vor, z.B. das Stearyltrimethylammoniumchlorid oder das Benzyl-di-(2-chloräthyl)-äthylammoniumbromid.

Die in der Formulierungstechnik gebräuchlichen Tenside sind u.a. in folgenden Publikationen beschrieben:

"Mc Cutcheon's Detergents and Emulsifiers Annual"
MC Publishing Corp., Ridgewood, New Jersey, 1979;
Dr. Helmut Stache "Tensid Taschenbuch", Carl Hanser Verlag München/Wien 1981.

Die pestiziden Zubereitungen enthalten - auf das Gewicht bezogen - in der Regel 0,1 bis 99 %, insbesondere 0,1 bis 95 %, eines Wirkstoffes der Formel I oder Kombinationen davon mit andern Insektiziden oder Akariziden, 1 bis 99,9 % eines festen oder flüssigen Zusatzstoffes und 0 bis 25 %, insbesondere 0,1 bis 20 %, eines Tensides. Während als Handelsware eher konzentrierte Mittel bevorzugt werden, verwendet der Endverbraucher in der Regel verdünnte Zubereitungen, die wesentlich geringere Wirkstoffkonzentrationen aufweisen.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

<u>Beispiel 1:</u> <u>Herstellung von 2-(4-Chlorphenyl)-5-(pyrid-3-yl)-4,5-dihydro-1,3,4-thiadiazol</u>

Es werden 39,2 g p-Chlorthiobenzoesäure-hydrazid in 350 ml Aethanol gelöst. Zu dieser Lösung werden unter Stickstoff 21,4 g Pyridin-3-aldehyd (gelöst in 20 ml Aethanol) zugetropft. Die Reaktionstemperatur wird durch Kühlung auf 20°C gehalten. Nach Beendigung der Zugabe wird während 1 Stunde bei Rückflusstemperatur nachgerührt und anschliessend das Reaktionsgemisch durch feinteilige Diatomeenerde filtriert. Das Filtrat wird auf Dreiviertel seines Anfangsvolumens eingeengt und das ausgefallene Produkt abfiltriert, mit Hexan gewaschen und getrocknet. Man erhält die Titelverbindung der Formel

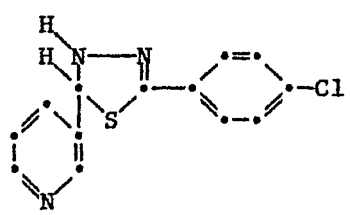

mit einem Smp. von 106-108°C (Verbindung Nr. 1.1).

<u>Beispiel 2:</u> <u>Herstellung von 2-(3-Nitrophenyl)-5-(pyrid-3-yl)-4,5-dihydro-1,3,5-thiadiazol</u>

Eine Lösung von 8,7 g Kaliumhydroxyd in 100 ml Aethanol wird mit Schwefelwasserstoff gesättigt. Zu dieser Lösung werden 8,7 g Kaliumhydroxyd in 100 ml Aethanol zugetropft. Anschliessend wird die erhaltene Lösung mit 22,4 g 1-Chlor-1-(3-nitrophenyl)-4-(3-pyridyl)-2,3-diazabutadien portionsweise unter Eiskühlung versetzt. Das Reaktionsgemisch wird während 1 Stunde bei Raumtemperatur nachgerührt, dann eingeengt und der Rückstand in Essigester aufgenommen. Die Essigester-Lösung wird zweimal mit Wasser und zweimal mit gesättigter Kochsalzlösung gewaschen, über Natriumsulfat getrocknet, filtriert und eingedampft.

Man erhält auf diese Weise die Titelverbindung der Formel

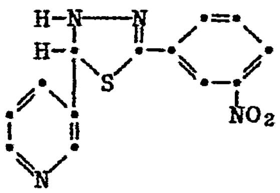

mit einem Schmelzpunkt von 109-111°C (Verbindung Nr. 1.2).

Entsprechend den vorstehend beschriebenen Arbeitsweisen werden die folgenden Verbindungen der Formel Ia) hergestellt.

| Verbindung Nr. | Stellung Pyridylrest | $R_1$ | $R_2$ | $R_3$ | physikal. Daten |
|---|---|---|---|---|---|
| 1.3 | 2 | H | 4-Cl | H | Smp. 91- 93°C |
| 1.4 | 4 | H | 4-Cl | H | Smp. 145-147°C |
| 1.5 | 3 | $-CH_3$ | 4-Cl | H | Smp. 119-120°C |
| 1.6 | 3 | H | H | H | Smp. 70- 73°C |
| 1.7 | 3 | $-CH_3$ | H | H | $n_D^{20}$ = 1,6450 |
| 1.8 | 4 | $-CH_3$ | 4-Cl | H | Smp. 123-125°C |
| 1.9 | 2 | $-CH_3$ | 4-Cl | H | Smp. 92- 93°C |
| 1.10 | 3 | H | 2-Cl | H | Smp. 125-126°C |
| 1.11 | 3 | H | 2-F | H | Smp. 78- 80°C |
| 1.12 | 3 | H | $4-CH_3$ | H | Smp. 118-119°C |
| 1.13 | 3 | H | $4-CF_3$ | H | Smp. 98-100°C |
| 1.14 | 3 | H | 3-F | H | Smp. 73- 75°C |
| 1.15 | 3 | H | 4-F | H | Smp. 103-105°C |
| 1.16 | 3 | H | 4-Br | H | Smp. 126-128°C |
| 1.17 | 3 | H | 3-Cl | H | Smp. 86- 87°C |
| 1.18 | 3 | H | $3-CH_3$ | H | Smp. 80- 82°C |
| 1.19 | 3 | H | $4-OCH_3$ | H | Smp. 93- 94°C |
| 1.20 | 3 | H | 3-Cl | 4-Cl | Smp. 104-108°C |

Wie vorstehend beschrieben sind auch die folgenden Verbindungen der Formel Ia) erhältlich:

| Stellung Pyridylrest | $R_1$ | $R_2$ | $R_3$ |
|---|---|---|---|
| 2 | n-$C_4H_9$ | H | H-$CH_3$ |
| 4 | H | H | 4-n-$C_4H_9$ |
| 2 | -$CH_3$ | 4-$OCH_3$ | H |
| 4 | -$C_2H_5$ | H | -$OCH_3$ |
| 2 | -n-$C_3H_7$ | 2-F | H |
| 4 | H | 2-Cl | 6-Cl |
| 3 | H | 2-Cl | 4-Cl |
| 3 | H | 2-Cl | 3-Cl |
| 3 | H | 3-F | 5-F |
| 3 | H | 3-F | 4-F |
| 3 | H | 2-F | 4-F |
| 3 | H | 2-F | 3-F |
| 3 | H | 4-$OCF_3$ | H |
| 3 | H | 4-$OCF_2CHFCF_3$ | H |
| 3 | H | 4-$OCHF_2$ | H |
| 3 | H | 4-CN | H |
| 3 | H | 4-O-Phenyl | H |
| 3 | H | 4-$CH_2$-Phenyl | H |
| 3 | H | 4-S-Phenyl | H |
| 3 | H | 3-O-Phenyl | H |
| 3 | H | 4-O-(3,5-$Cl_2$-Pyridyl) | H |
| 3 | H | 4-$C_2H_5$ | H |
| 3 | H | 3-Cl | 5-Cl |

Daneben sind, wie vorstehend beschrieben die folgenden, der Formel I entsprechenden Verbindungen erhältlich:

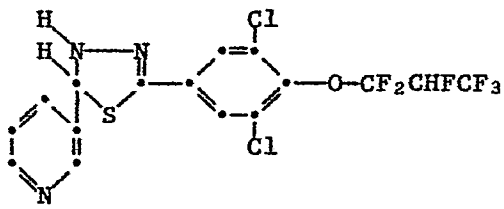

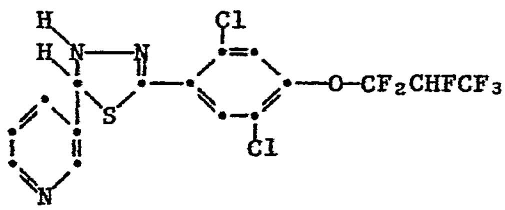

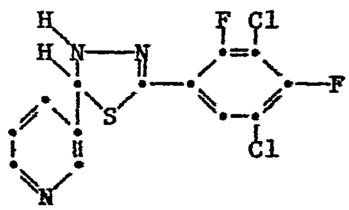

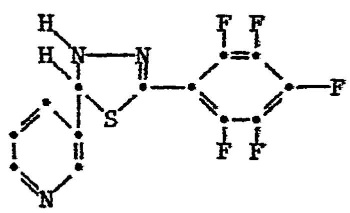

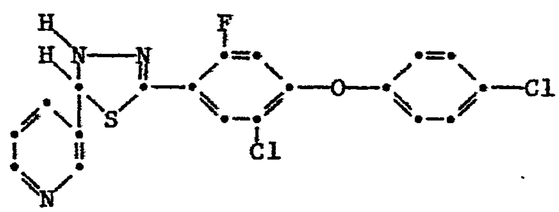

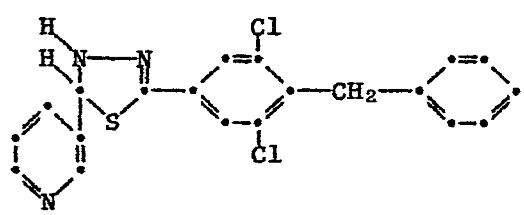

Wie vorstehend beschrieben sind ferner die folgenden Salze einer Verbindung der Formel Ia erhältlich:

| Verbindung Nr. | Stellung Pyridylrest | $R_1$ | $R_2$ | $R_3$ | salzbildende Säure | Physikal. Daten [°C] |
|---|---|---|---|---|---|---|
| 2.1 | 3 | H | 4-Cl | H | Oxalsäure | Smp. 200-201 |
| 2.2 | 3 | H | 4-Cl | H | HBr | Smp. 232-234 |
| 2.3 | 3 | H | 4-Cl | H | p-Toluolsulfonsäure | Smp. 261-262 |
| 2.4 | 3 | H | 4-Cl | H | Benzoesäure | Smp. 70- 72 |
| 2.5 | 3 | H | 4-Cl | H | HCl | Smp. 192-193 |
| 2.6 | 3 | H | 4-Cl | H | HBr | Smp. 228-229 |

Beispiel 3:

Formulierungen für Wirkstoffe der Formeln I bzw. Ia) gemäss Beispiel 1 und 2 resp. Kombinationen dieser Wirkstoffe mit andern Insektiziden oder Akariziden (% = Gewichtsprozent):

| 1. Spritzpulver | a) | b) | c) |
|---|---|---|---|
| Wirkstoff oder Wirkstoffkombination | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyäthylenglykoläther (7-8 Mol AeO) | - | 2 % | - |
| Hochdisperse Kieselsäure | 5 % | 10 % | 10 % |
| Kaolin | 62 % | 27 % | - |

Der Wirkstoff oder die Wirkstoffkombination wird mit den Zusatzstoffen vermischt und in einer geeigneten Mühle gut vermahlen.

Man erhält Spritzpulver, die sich mit Wasser zu Suspensionen jeder gewünschten Konzentration verdünnen lassen.

2. Emulsions-Konzentrat

| | |
|---|---|
| Wirkstoff oder Wirkstoffkombination | 10 % |
| Octylphenolpolyäthylenglykoläther (4-5 Mol AeO) | 3 % |
| Ca-Dodecylbenzolsulfonat | 3 % |
| Ricinusölpolyglykoläther (36 Mol AeO) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

| 3. Stäubemittel | a) | b) |
|---|---|---|
| Wirkstoff oder Wirkstoffkombination | 5 % | 8 % |
| Talkum | 95 % | - |
| Kaolin | - | 92 % |

Man erhält anwendungsfertige Stäubemittel, indem der Wirkstoff mit dem Träger vermischt und auf einer geeigneten Mühle vermahlen wird.

4. Extruder-Granulat

| | |
|---|---|
| Wirkstoff oder Wirkstoffkombination | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert, granuliert und anschliessend im Luftstrom getrocknet.

5. Umhüllungs-Granulat

| | |
|---|---|
| Wirkstoff oder Wirkstoffkombination | 3 % |
| Polyäthylenglykol (MG 200) | 3 % |
| Kaolin | 94 % |

Der fein gemahlene Wirkstoff oder die Wirkstoffkombination wird in einem Mischer auf das mit Polyäthylenglykol angefeuchtete Kaolin gleichmässig aufgetragen. Auf diese Weise erhält man staubfreie Umhüllungs-Granulate.

6. Suspensions-Konzentrat

| | | |
|---|---|---|
| Wirkstoff oder Wirkstoffkombination | 40 | % |
| Aethylenglykol | 10 | % |
| Nonylphenolpolyäthylenglykoläther (15 Mol AeO) | 6 | % |
| Na-Ligninsulfonat | 10 | % |
| Carboxymethylcellulose | 1 | % |
| 37%ige wässrige Formaldehyd-Lösung | 0,2 | % |
| Silikonöl in Form einer 75%igen wässrigen Emulsion | 0,8 | % |
| Wasser | 32 | % |

Der fein gemahlene Wirkstoff oder die Wirkstoffkombination wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Beispiel 4: Wirkung gegen Musca domestica

Je 50 g frisch zubereitetes CSMA-Nährsubstrat für Maden werden in Becher eingewogen. Von einer 1 Gew.-%igen acetonischen Lösung des betreffenden Wirkstoffes wird eine bestimmte Menge auf das in den Bechern befindliche Nährsubstrat pipettiert, so dass sich eine Wirkstoffkonzentration von 400 ppm ergibt. Nach dem Durchmischen des Substrates lässt man das Aceton mindestens 20 Stunden lang verdampfen.

Dann werden pro Wirkstoff je 25 eintägige Maden von Musca domestica in die das so behandelte Nährsubstrat enthaltenden Becher gegeben. Nachdem sich die Maden verpuppt haben, werden die gebildeten Puppen durch Ausschwemmen mit Wasser von dem Substrat abgetrennt und in mit Siebdeckeln verschlossenen Gefässen deponiert.

Die pro Ansatz ausgeschwemmten Puppen werden gezählt (toxischer Einfluss des Wirkstoffes auf die Madenentwicklung). Dann wird nach 10 Tagen die Anzahl der aus den Puppen geschlüpften Fliegen bestimmt.

Verbindungen der Formel I bzw. Ia) gemäss Beispiel 1 und 2 zeigen gute Wirkung im obigen Test.

Beispiel 5: Wirkung gegen Lucilia sericata (Larven)
Zu 9 ml eines Zuchtmediums wird bei 50°C 1 ml einer 0,5 % Aktivsubstanz enthaltenden wässrigen Zubereitung gegeben. Nun werden ca. 30 frisch geschlüpfte Lucilia sericata-Larven zum Zuchtmedium gegeben. Nach 48 und 96 Stunden wird die insektizide Wirkung durch Ermittlung der Abtötungsrate festgestellt.

Verbindungen der Formel I bzw. Ia) gemäss Beispiel 1 und 2 zeigen in diesem Test gute Wirkung gegen Lucilia sericata.

Beispiel 6: Wirkung gegen Aedes aegypti (Larven)
Auf die Oberfläche von 150 ml Wasser, das sich in einem Behälter befindet, wird so viel einer 0,1%igen acetonischen Lösung des Wirkstoffes pipettiert, dass eine Konzentration von 200 ppm erhalten wird. Nach Verdunsten des Acetons wird der Behälter mit 30 bis 40 2-tägigen Aedes-Larven beschickt. Nach 2 und 7 Tagen wird die Mortalität geprüft.

Verbindungen der Formel I bzw. Ia) gemäss Beispiel 1 und 2 zeigen gute Wirkung im obigen Test.

Beispiel 7: Kontaktwirkung auf Aphis craccivora
In Töpfen angezogene 4-5 Tage alte Erbsenkeimlinge (Vicia faba) werden vor Versuchsbeginn mit je ca. 200 Individuen der Spezies Aphis craccivora besiedelt. Die so behandelten Pflanzen werden 24 Stunden später mit einer wässrigen Zubereitung enthaltend die zu prüfenden Verbindung bis zur Tropfnässe direkt besprüht. Man

verwendet pro Test-Verbindung zwei Pflanzen. Eine Auswertung der erzielten Abtötungsrate erfolgt nach weiteren 24 und 72 Stunden. Der Versuch wird bei 21-22°C und einer rel. Luftfeuchtigkeit von etwa 55 % durchgeführt.

Eine Mortalität von 80-100 % in diesem Test ergibt die Verbindung Nr. 1.1 gemäss Beispiel 1 bei 3 ppm. Die gleiche Wirkung zeigen bei 400 ppm die Verbindungen Nr. 1.6, 1.11, 2.1, 2.2, 2.3, 2.4 und 2.5.

Beispiel 8: Systemische Wirkung auf Aphis craccivora

Bewurzelte Bohnenpflanzen werden in Töpfe, welche 600 ccm Erde enthalten, verpflanzt. Anschliessend giesst man 50 ml einer Zubereitung der zu prüfenden Verbindungen (erhalten aus einem 25 %igen Spritzpulver) in einer Konzentration von 800 ppm direkt auf die Erde in den Töpfen.

Nach 24 Stunden werden auf die oberirdischen Pflanzenteile Blattläuse der Spezies Aphis craccivora gesetzt und die Pflanzen mit einem Plastikzylinder überstülpt, um die Läuse vor einer eventuellen direkten Kontakt- oder Gaswirkung der Testsubstanz zu schützen.

Die Auswertung der erzielten Abtötung erfolgt 48 und 72 Stunden nach Versuchsbeginn. Pro Testsubstanz werden zwei Pflanzen, je eine in einem separaten Topf, verwendet. Der Versuch wird bei 25°C und ca. 70 % relativer Luftfeuchtigkeit durchgeführt.

Verbindungen der Formel I bzw. Ia) gemäss Beispiel 1 und 2 zeigen gute Wirkung in diesem Test.

Beispiel 9: Kontaktwirkung auf Myzus persicae

Etwa 4 bis 5 Tage alte, in Wasser angezogene Erbsenkeimlinge (Vicia faba) werden vor Versuchsbeginn jeweils mit ca. 200 Individuen der Spezies Myzus persicae besiedelt. Die so behandelten Pflanzen werden 24 Stunden später mit einer wässrigen Suspension enthaltend bis zu 200 ppm der zu prüfenden Verbindung bis zur Tropfnässe direkt besprüht. Man verwendet pro Testsubstanz zwei Pflanzen. Eine

Auswertung der erzielten Abtötungsrate erfolgt 24 und 72 Stunden nach Applikation. Der Versuch wird bei 21-22°C und etwa 60 % relativer Luftfeuchtigkeit durchgeführt.

Verbindung Nr. 1 gemäss Beispiel 1 ergibt bei 200 ppm, Verbindung Nr. 2.5 bei 3 ppm und die Verbindungen Nr. 2.1, 2.2, 2.3 und 2.4 ergeben bei 50 ppm eine 80-100 %ige Mortalität in diesem Test.

Beispiel 10: Systemische Wirkung auf Myzus persicae

Bewurzelte Kohlpflanzen im 4- bis 5-Blatt-Stadium werden in Töpfe, welche 60 ccm Erde enthalten, verpflanzt. Ansschliessend werden 50 ml einer wässrigen Formulierung der zu prüfenden Verbindung (erhalten aus einem 25%igen Spritzpulver) jeweils in einer Konzentration von 800 ppm direkt auf die Erde aufgegossen.

Nach 24 Stunden werden auf die oberirdischen Pflanzenteile der behandelten Pflanzen Blattläuse der Spezies Myzus persicae gesetzt und die Pflanzen mit Plastikzylindern überdeckt, um die Blattläuse vor einer eventuellen direkten Kontakt- oder Gaswirkung der Testsubstanz zu schützen.

Die Auswertung der erzielten %-Abtötung erfolgt 48 Stunden nach Versuchsbeginn. Pro Testsubstanz werden zwei Pflanzen, je eine in separaten Töpfen, verwendet. Der Versuch wird bei ca. 25°C und 60 % relativer Luftfeuchtigkeit durchgeführt.

Verbindungen der Formel I bzw. Ia) gemäss Beispiel 1 und 2 zeigen gute Wirkung in obigem Test.

Beispiel 11: Blattpenetrations-Wirkung auf Aphis craccivora

In etwa 8 cm hohe Kunststoffbecher (Durchmesser etwa 6 cm) wird ein passender kleiner Zweig von Vicia faba gelegt, der mit Blattläusen der Spezies Aphis craccivora stark infiziert ist. Der Becher wird mit einem Kunststoffdeckel, der in der Mitte eine ausgestanzte Oeffnung von 2 cm Durchmesser aufweist, bedeckt. Auf die in dem Deckel befindliche Oeffnung wird ein Blatt einer Vicia faba-Pflanze

gelegt, ohne dieses Blatt von der eingetopften Pflanze abzutrennen. Das Blatt wird dann mit einem zweiten Lochdeckel auf dem Becher über der Oeffnung des ersten Deckels fixiert. Von der Unterseite her, d.h. durch die Oeffnung des ersten Deckels hindurch, infizieren nun die in dem Becher befindlichen Blattläuse das obenliegende Blatt der Futterpflanze. Auf der Oberseite wird auf das Blatt eine wässrige Zubereitung des zu prüfenden Wirkstoffes in einer Konzentration von 400 ppm mittels eines Pinsels gleichmässig aufgetragen. Es wird geprüft, ob die einseitig auf die Oberseite des Blattes der Futterpflanze aufgetragene Testsubstanz in genügender Menge durch das Blatt hindurch auf dessen Unterseite diffundiert, um dort saugende Blattläuse abzutöten.

Der Versuch wird bei etwa 20°C und 60 % relativer Luftfeuchtigkeit durchgeführt. Die Auswertung auf %-Mortalität erfolgt 48 Stunden nach Wirkstoffapplikation.

Verbindungen der Formel I bzw. Ia) gemäss Beispiel 1 und 2 zeigen gute Wirkung in obigem Test.

<u>Beispiel 12: Frassgift-Wirkung auf Laodelphax striatellus und Nilaparvata lugens (Nymphen);</u>

Der Test wird an wachsenden Pflanzen durchgeführt. Dazu werden jeweils 4 Reispflanzen (Dicke des Stengels 8 mm) mit einer Höhe von ca. 20 cm in Töpfe (Durchmesser von 8 cm) eingepflanzt.

Die Pflanzen werden auf einem Drehteller mit 100 ml einer acetonischen Lösung enthaltend 800 ppm des jeweiligen Wirkstoffes besprüht. Nach dem Antrocknen des Spritzbelages erfolgt die Besiedlung jeder Pflanze mit je 20 Nymphen der Testtiere im dritten Stadium. Um die Zikaden am Entweichen zu hindern, wird über die besiedelten Pflanzen jeweils ein beidseitig offener Glaszylinder gestülpt und dieser mit einem Gaze-Deckel abgedeckt. Die Nymphen werden bis zum Erreichen des folgenden Entwicklungsstadiums über 10 Tage an der behandelten Pflanze gehalten. Die Auswertung auf %-Mortalität erfolgt 1, 4 und 8 Tage nach der Behandlung.

Verbindungen der Formel I bzw. Ia) gemäss Beispiel 1 und 2 zeigen gute Wirkung in diesem Test.

Beispiel 13: Ovizide Wirkung auf Laodelphax striatellus und Nilaparvata lugens;
Der Test wird an wachsenden Pflanzen durchgeführt. Jeweils 4 Reispflanzen (Dicke des Stengels 8 mm, Höhe ca. 20 cm) werden in Töpfe (Durchmesser von 8 cm) eingepflanzt.

Die Pflanzen werden auf einem Drehteller mit 100 ml einer acetonischen Lösung enthaltend 400 ppm des jeweiligen Wirkstoffes besprüht. Nach dem Antrocknen des Spritzbelages wird jede Pflanze mit 3 adulten Weibchen besiedelt. Um die Tiere am Entweichen zu hindern, wird über jede besiedelte Pflanze ein Glaszylinder gestülpt und dieser mit einem Gaze-Deckel abgedeckt. Die Weibchen bleiben 4 Tage zur Eiablage auf der behandelten Pflanze und werden dann entfernt.

Etwa 8 Tage nach Besiedelung schlüpfen die jungen Zikaden, und es erfolgt die Auszählung. Aus dem Vergleich der Anzahl geschlüpfter Larven auf den behandelten Pflanzen zu den auf unbehandelten Kontrollpflanzen geschlüpften Tieren wird die prozentuale Mortalität bestimmt.

Verbindungen der Formel I bzw. Ia) gemäss Beispiel 1 und 2 zeigen in obigem Test gute ovizide Wirkung.

Patentansprüche für die Vertragsstaaten: DE, GB, FR, CH + LI, IT, NL, BE

1. Verbindung der Formel I

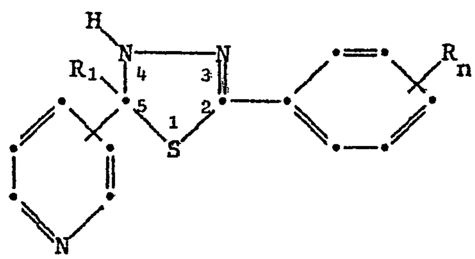

(I),

worin

$R_1$ Wasserstoff oder $C_1$-$C_4$-Alkyl;

R Wasserstoff, $C_1$-$C_4$-Alkyl, Halogen, Nitro, Cyano, $C_1$-$C_4$-Alkoxy, $C_1$-$C_4$-Alkylthio, $C_1$-$C_4$-Halogenalkyl mit 1 bis 9 Halogenatomen, $C_1$-$C_4$-Halogenalkoxy mit 1 bis 9 Halogenatomen, $C_1$-$C_4$-Halogenalkylthio mit 1 bis 9 Halogenatomen, Phenyl, substituiertes Phenyl, Phenylalkyl, substituiertes Phenylalkyl, Phenoxy, substituiertes Phenoxy, Phenylthio, substituiertes Phenylthio, Pyridyloxy oder substituiertes Pyridyloxy bedeuten und

n für eine Zahl von 1 bis 5 steht; sowie die Salze und optischen Isomeren einer Verbindung der Formel I.

2. Verbindung der Formel I, gemäss Anspruch 1, dadurch gekennzeichnet, dass

$R_1$ Wasserstoff oder $C_1$-$C_4$-Alkyl;

R Wasserstoff, $C_1$-$C_4$-Alkyl, Halogen, Nitro, Cyano, $C_1$-$C_4$-Alkoxy, $C_1$-$C_4$-Alkylthio, $C_1$-$C_4$-Halogenalkyl mit 1 bis 9 Halogenatomen, $C_1$-$C_4$-Halogenalkoxy mit 1 bis 9 Halogenatomen oder $C_1$-$C_4$-Halogenalkylthio mit 1 bis 9 Halogenatomen bedeuten und

n für eine Zahl von 1 bis 5 steht; sowie die Salze und optischen Isomeren einer Verbindung der Formel I.

3. Verbindung gemäss Anspruch 1 oder 2, dadurch gekennzeichnet, dass n für 1 oder 2 steht.

4. Verbindung gemäss Anspruch 3 der Formel Ia)

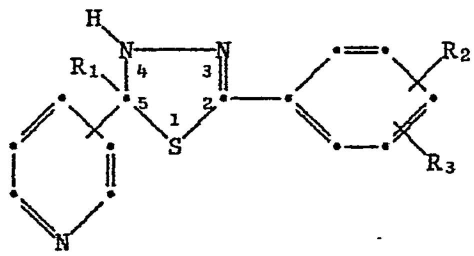

(Ia),

dadurch gekennzeichnet, dass
$R_1$ Wasserstoff oder $C_1$-$C_4$-Alkyl;
$R_2$ und $R_3$ unabhängig voneinander Wasserstoff, Halogen, $C_1$-$C_4$-Alkyl, Trifluormethyl, $C_1$-$C_4$-Alkoxy oder Nitro
bedeuten; sowie die Salze einer Verbindung der Formel Ia).

5. Verbindung der Formel Ia) gemäss Anspruch 4, dadurch gekennzeichnet, dass
$R_1$ Wasserstoff, Methyl oder Aethyl;
$R_2$ und $R_3$ unabhängig voneinander Wasserstoff oder in 2- und/oder 4-Stellung befindliches Halogen
bedeuten.

6. Verbindung der Formel Ia) gemäss einem der Ansprüche 4 oder 5, dadurch gekennzeichnet, dass $R_3$ Wasserstoff bedeutet.

7. Verbindung gemäss einem der Ansprüche 1 bis 6, dadurch gekennzeichnet, dass $R_1$ Wasserstoff bedeutet.

8. Verbindung gemäss einem der Ansprüche 1 bis 7, dadurch gekennzeichnet, dass in den Formeln I bzw. Ia) der Pyridylrest mit seiner 3-Stellung an die 5-Stellung des 4,5-Dihydro-1,3,4-thiadiazolringes gebunden ist.

9. Verbindung gemäss Anspruch 6 der Formel

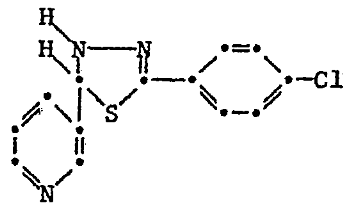

.

10. Verbindung gemäss Anspruch 6 der Formel

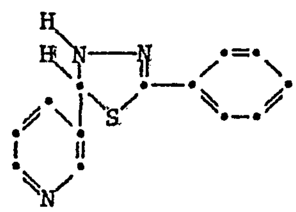

.

11. Verbindung gemäss Anspruch 6 der Formel

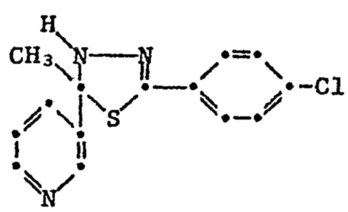

.

12. Verfahren zur Herstellung einer Verbindung der Formeln I bzw. Ia) gemäss einem der Ansprüche 1 bis 11, dadurch gekennzeichnet, dass man

a) eine Verbindung der Formel II

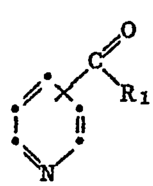

(II)

mit einer Verbindung der Formel III

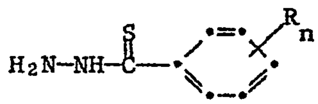

(III), oder

b) eine Verbindung der Formel IV

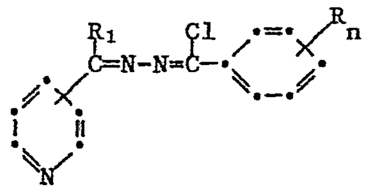

(IV) ,

mit einem Sulfid umsetzt, wobei $R_1$, R und n die unter Anspruch 1 bzw. 4 angegebenen Bedeutungen haben, und dass man gegebenenfalls eine erhaltene Verbindung der Formel I bzw. Ia in eines ihrer Salze überführt oder in ihre optischen Isomeren trennt.

13. Verbindung der Formel IV

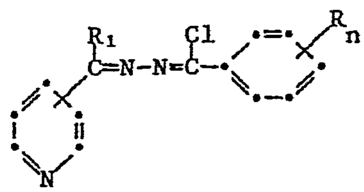

(IV) ,

worin $R_1$, R und n die in Anspruch 1 angegebenen Bedeutungen haben.

14. Schädlingsbekämpfungsmittel, welches als aktive Komponente eine Verbindung gemäss einem der Ansprüche 1 bis 11 zusammen mit geeigneten Trägern und/oder anderen Zuschlagstoffen enthält.

15. Verwendung einer Verbindung gemäss einem der Ansprüche 1 bis 11 zur Bekämpfung von Insekten und Vertretern der Ordnung Akarina an Tieren und Pflanzen.

16. Verwendung gemäss Anspruch 15 zur Bekämpfung von pflanzenschädigenden Insekten.

17. Verwendung gemäss Anspruch 16 zur Bekämpfung von pflanzenschädigenden saugenden Insekten.

18. Verfahren zur Bekämpfung von Insekten und Vertretern der Ordnung Akarina, dadurch gekennzeichnet, dass man die Schädlinge bzw. deren verschiedene Entwicklungsstadien und/oder ihren Aufenthaltsort mit

einer pestizid wirksamen Menge einer Verbindung der Formel I bzw. Ia) gemäss einem der Ansprüche 1 bis 11 oder mit einem Mittel enthaltend neben Zusatz- und Trägerstoffen eine pestizid wirksame Menge dieser Verbindung, in Kontakt bringt oder behandelt.

FO 7.5/EIC/cw*/sm*/we*

Patentansprüche für den Vertragsstaat: AT

1. Verfahren zur Herstellung einer Verbindung der Formel I

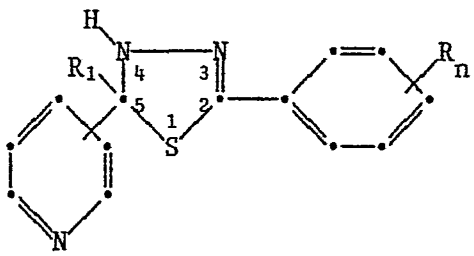

(I),

worin

$R_1$ Wasserstoff oder $C_1$-$C_4$-Alkyl;

R Wasserstoff, $C_1$-$C_4$-Alkyl, Halogen, Nitro, Cyano, $C_1$-$C_4$-Alkoxy, $C_1$-$C_4$-Alkylthio, $C_1$-$C_4$-Halogenalkyl mit 1 bis 9 Halogenatomen, $C_1$-$C_4$-Halogenalkoxy mit 1 bis 9 Halogenatomen, $C_1$-$C_4$-Halogenalkylthio mit 1 bis 9 Halogenatomen, Phenyl, substituiertes Phenyl, Phenylalkyl, substituiertes Phenylalkyl, Phenoxy, substituiertes Phenoxy, Phenylthio, substituiertes Phenylthio, Pyridyloxy oder substituiertes Pyridyloxy bedeuten und

n für eine Zahl von 1 bis 5 steht; sowie der Salze und optischen Isomeren einer Verbindung der Formel I, dadurch gekennzeichnet, dass man

a) eine Verbindung der Formel II

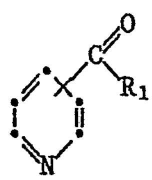

(II)

mit einer Verbindung der Formel III

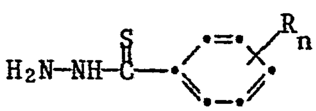

(III), oder

b) eine Verbindung der Formel IV

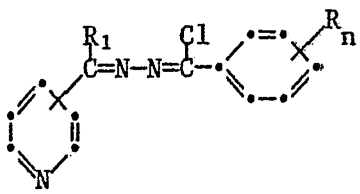

(IV),

mit einem Sulfid umsetzt, wobei $R_1$, R und n die vorstehend angegebenen Bedeutungen haben, und dass man gegebenenfalls eine erhaltene Verbindung der Formel I in eines ihrer Salze überführt oder in ihre optischen Isomeren trennt.

2. Verfahren zur Herstellung einer Verbindung der Formel I gemäss Anspruch 1, worin

$R_1$ Wasserstoff oder $C_1$-$C_4$-Alkyl;

R Wasserstoff, $C_1$-$C_4$-Alkyl, Halogen, Nitro, Cyano, $C_1$-$C_4$-Alkoxy, $C_1$-$C_4$-Alkylthio, $C_1$-$C_4$-Halogenalkyl mit 1 bis 9 Halogenatomen, $C_1$-$C_4$-Halogenalkoxy mit 1 bis 9 Halogenatomen oder $C_1$-$C_4$-Halogenalkylthio mit 1 bis 9 Halogenatomen bedeuten und

n für eine Zahl von 1 bis 5 steht; sowie deren Salze und optischen Isomeren.

3. Verfahren zur Herstellung einer Verbindung der Formel I gemäss Anspruch 1 oder 2, worin n für 1 oder 2 steht.

4. Verfahren gemäss Anspruch 3 zur Herstellung einer Verbindung der Formel Ia)

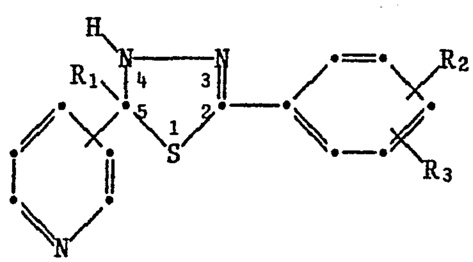

(Ia),

worin

$R_1$ Wasserstoff oder $C_1$-$C_4$-Alkyl;

$R_2$ und $R_3$ unabhängig voneinander Wasserstoff, Halogen, $C_1$-$C_4$-Alkyl, Trifluormethyl, $C_1$-$C_4$-Alkoxy oder Nitro bedeuten; sowie der Salze einer Verbindung der Formel Ia).

5. Verfahren zur Herstellung einer Verbindung der Formel Ia) gemäss Anspruch 4, worin

$R_1$ Wasserstoff, Methyl oder Aethyl;

$R_2$ und $R_3$ unabhängig voneinander Wasserstoff oder in 2- und/oder 4-Stellung befindliches Halogen

bedeuten.

6. Verfahren zur Herstellung einer Verbindung der Formel Ia) gemäss einem der Ansprüche 4 oder 5, worin $R_3$ Wasserstoff bedeutet.

7. Verfahren zur Herstellung einer Verbindung gemäss einem der Ansprüche 1 bis 6, worin $R_1$ Wasserstoff bedeutet.

8. Verfahren zur Herstellung einer Verbindung gemäss einem der Ansprüche 1 bis 7, wobei in den Formeln I bzw. Ia) der Pyridylrest mit seiner 3-Stellung an die 5-Stellung des 4,5-Dihydro-1,3,4-thiadiazolringes gebunden ist.

9. Verfahren zur Herstellung einer Verbindung gemäss Anspruch 6 der Formel

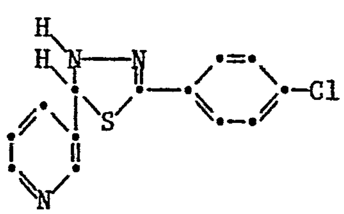

10. Verfahren zur Herstellung einer Verbindung gemäss Anspruch 6 der Formel

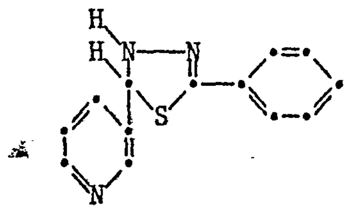

11. Verfahren zur Herstellung einer Verbindung gemäss Anspruch 6 der Formel

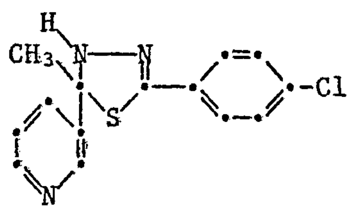

12. Verfahren zur Herstellung einer Verbindung der Formel IV gemäss Anspruch I

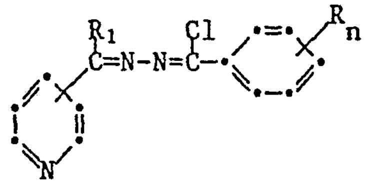

(IV) ,

dadurch gekennzeichnet, dass man eine Verbindung der Formel V

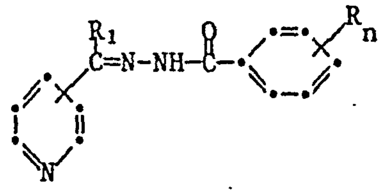

(V)

mit $SOCl_2$ umsetzt, wobei in den Formeln IV und V $R_1$, R und n die in Anspruch 1 angegebenen Bedeutungen haben.

13. Schädlingsbekämpfungsmittel, welches als aktive Komponente eine Verbindung gemäss einem der Ansprüche 1 bis 11 zusammen mit geeigneten Trägern und/oder anderen Zuschlagstoffen enthält.

14. Verwendung einer Verbindung gemäss einem der Ansprüche 1 bis 11 zur Bekämpfung von Insekten und Vertretern der Ordnung Akarina an Tieren und Pflanzen.

15. Verwendung gemäss Anspruch 14 zur Bekämpfung von pflanzenschädigenden Insekten.

16. Verwendung gemäss Anspruch 15 zur Bekämpfung von pflanzenschädigenden saugenden Insekten.

17. Verfahren zur Bekämpfung von Insekten und Vertretern der Ordnung Akarina, dadurch gekennzeichnet, dass man die Schädlinge bzw. deren verschiedene Entwicklungsstadien und/oder ihren Aufenthaltsort mit einer pestizid wirksamen Menge einer Verbindung der Formel I bzw. Ia) gemäss einem der Ansprüche 1 bis 11 oder mit einem Mittel enthaltend neben Zusatz- und Trägerstoffen eine pestizid wirksame Menge dieser Verbindung, in Kontakt bringt oder behandelt.

FO 7.5/EIC/ga*